# EUROPEAN PATENT SPECIFICATION

(11) **EP 1 997 930 B1**
(45) Date of publication and mention of the grant of the patent: **21.03.2012**
(21) Application number: 07253060.3
(22) Date of filing: 03.08.2007
(51) Int. Cl.: C23C 14/00, C23C 14/34, A61N 1/00

(54) **Method for producing a coating with improved adhesion**
Verfahren zur Herstellung einer Beschichtung mit verbesserter Haftung
Procédé pour la fabrication d'un revêtement à adhésion améliorée

(30) Priority: 29.05.2007 US 754601
(43) Date of publication of application: 03.12.2008
(73) Proprietor: Pulse Technologies, Inc., Quakertown, PA 18951 (US)
(72) Inventor: Fisk, Andrew, Philadelphia Pennsylvania 19118 (US)
(74) Representative: Nash, David Allan

(56) References cited:
- WO-A-03/107385
- US-A- 5 543 019
- US-A1- 2001 030 125
- LOU ET AL: "Effect of deposition conditions on the characteristics of reactively sputtered titanium nitride films" SURFACE AND COATINGS TECHNOLOGY, ELSEVIER, AMSTERDAM, NL, vol. 90, no. 1-2, 15 March 1997 (1997-03-15), pages 123-127, XP022390347 ISSN: 0257-8972
- THORNTON J A: "INFLUENCE OF APPARATUS GEOMETRY AND DEPOSITION CONDITIONS ON THE STRUCTURE AND TOPOGRAPHY OF THICK SPUTTERED COATINGS" JOURNAL OF VACUUM SCIENCE AND TECHNOLOGY, NEW YORK, NY, US, vol. 11, no. 4, 1 July 1974 (1974-07-01), pages 666-670, XP000992157 ISSN: 0022-5355

## Description

### Field of the Invention

This invention relates generally to coatings for medical devices, and, in particular, to coatings for devices intended to be permanently implanted into the human body.

### Background of the Invention

Active implantable devices are typically electrodes used for the stimulation of tissue or the sensing of electrical bio-rhythms. Typically, the electrical performance of implantable electrodes can be enhanced by applying a coating to the external surfaces which are in contact with tissues inside the body. It is known that the application of a high surface area or highly porous coating to an implantable electrode increases the double layer capacitance of the electrode and reduces the after-potential polarization, thereby increasing device battery life, or allowing for lower capture thresholds and improved sensing of certain electrical signals, such as R and P waves.

Such coatings, in addition to a having a large surface area and being biocompatible and corrosion resistant in bodily fluids, must strongly adhere to the substrate (the electrode surface) and have good abrasion resistance, showing no signs of flaking during post-coating assembly and use. Adhesion of an electrode coating is of critical interest since the flaking of a coating during implant can cause infection and flaking of the coating post-implant can cause a sudden increase in the charge required to stimulate tissue.

Coatings having large surface areas are produced as porous deposits having morphologies described as columnar or cauliflower in structure. Such coatings may be deposited on the surface of the electrode by any means well known in the art, such as by physical vapor deposition or sputtering. It is known in the prior art that a decrease in the energy of the system during the deposition of the coating will inhibit the surface mobility of the deposited material and therefore increase the porosity. This porosity is a function of the physical shadowing which occurs as a result of the coating species condensing upon arrival at the surface rather then being mobile on the surface and nucleating new sites between already formed nucleation sites.

The presently known method for producing a coating having a high porosity is dependent on decreasing the surface mobility of the condensing material and is accomplished by reducing the energy at the surface. This can be accomplished several ways. The deposition pressure can be increased, thereby increasing the number of atomic collisions and reducing the energy of the coating flux. The angle of incidence of coating flux can be decreased, such that the nucleating material does not impart excess energy to the surface, which would increase surface mobility. Lastly, the substrate temperature can be decreased, which also results in a decrease in surface mobility.

Often the method to produce a porous coating for medical implantable electrodes calls for a combination of two or more of the aforementioned methods. All of the methods have the effect of reducing the overall energy in the system, so that limited surface diffusion takes place. By limiting the surface diffusion, the depositing species condenses quickly on the surface, forming new nucleation sites, which causes an increase in porosity. This type of coating is therefore characterized by highly open, fibrous, dendrite grains with a poorly resolved inner structure.

Because of this structure, porous coatings are generally brittle, exhibit poor abrasion resistance and have an adhesive strength much lower then that normally expected from the chemical bond formed during vacuum processing.

The desirable characteristics of the coating, those being high double layer capacitance of the electrode and a low after-potential polarization effect, are enhanced when the surface area of the coating is increased. Although having a porous surface is one way to increase surface area, the undesirable result of a brittle coating having poor adhesion strength and abrasion resistance is obtained. It would therefore be advantageous to be able to produce a coating having a high surface area without the drawbacks of the prior art porous coatings, while still retaining the desirable characteristics.

Surface and Coatings Technology 90 (1997) 123-127 is a paper entitled Effect of deposition conditions on the characteristics of reactively sputtered titanium nitride films.

US 2001/0030125 relates to PVD deposition of titanium and titanium nitride layers in the same chamber without use of a collimator or a shutter.

WO 03/107385 relates to a device for coating, substrates by physical vapour deposition, using a hollow discharge method.

US 5,543,019 relates to a method of coating medical devices and a device coated thereby.

### Summary of the Invention

The present invention meets these objectives by disclosing a process for producing a coating which enhances the electrical and mechanical performance of an implantable medical electrode while mitigating the undesirable effects associated with prior art porous surfaces.

In an aspect, the present invention provides a method for producing a coating on a substrate using a physical vapor deposition comprising the steps of:
a. providing one or more targets composed of a primary metallic component
b. raising the temperature of said substrate to between 20% and 50% of the melting point of said primary metallic component;
c. positioning said one or more target with respect to said substrate such that a percentage of atoms of said primary component reach said substrate at a normal angle;
d. mixing a secondary reactive component with an inert gas to form a plasma, wherein said secondary reactive component is introduced with the inert gas in a flow ratio of 1:1 to 1.25:1;
e. wherein said primary metallic component and said secondary reactive component react to form a surface having pyramidal crystals defined thereon,
wherein said primary metallic component is allowed to condense on said substrate in the absence of said secondary reactive component to form an intermix layer wherein the atoms of said primary metallic component an the atoms of said substrate are intermixed, and further wherein said secondary reactive component is introduced after the formation of said intermix layer,
the thickness of the coating is 2.5 microns to 8 microns, and
plasma flux strikes the surface of the substrate at a normal angle.

In a further aspect, the present invention provides an implantable medical electrode having a coating thereon comprising a zone 2 microstructure composed of a primary metallic component and a secondary reactive component, said surface having crystals with a [1, 1, 1] structure defined thereon, wherein an intermix layer has been formed between the surface of said electrode and said coating, said intermix layer composed primarily of said primary metallic component intermixed at the surface of said electrode with atoms of the material of which said electrode is composed the coating and intermix layer formable by the method of any one of claims 1 to 9, such that the thickness of the coating is 2.5 microns to 8 microns.

During the coating process, the substrate is held at a temperature which allows surface diffusion prior to the solidification of the coating condensate. This tends to result in larger or more diffuse nucleation sites, or may eliminate the nucleation sites in some instances. The surface diffusion also promotes an intermix layer where the electrode base material is in alloy or solid solution with the metallic constituent of the condensate.

In the preferred method, the substrate temperature is held between approximately 20% and 40% of the melting point of the metallic coating species. This elevated temperature promotes diffusion and intermixing of the materials.

The presence of surface diffusion has a beneficial effect on adhesion. Because the metallic coating constituent is diffusing with the substrate, the bonding between the substrate and the coating consists of a intermixed diffusion layer. This is in contrast to the bonding in the current art where there is limited surface diffusion and therefore no diffusion layer.

The presence of surface diffusion also has a beneficial effect on abrasion resistance, because the newly deposited condensate is allowed to diffuse into the condensate which has already been deposited. This diffusion also tends to limit the number of nucleation sites within the coating and promotes the growth of continuous columns of condensate. These continuous columns promote good abrasion resistance, especially when compared to porous coatings of the current art having multiple nucleation sites within the coating thickness.

A second aspect of the invention involves the surface of the coating, which has pyramidal structures formed thereon, thereby providing an extremely high surface area, but with reduced porosity. A crystal structure direction which results in this structure must be thermodynamically preferred. For the cubic systems, this crystal direction is [1, 1, 1], however the [1, 1, 1] direction is not commonly thermodynamically preferred at temperatures above 20% of the melting point of the metallic coating species.

Therefore, to promote the growth of the [1, 1, 1] structure, a secondary constituent which is reactive with the metallic constituent is added to the process. The addition of the secondary reactive constituent to the coating process results in a loss of energy during the surface diffusion process. As the metallic constituent is condensing, the high surface diffusion energy allows the secondary reactive constituent to diffuse into the metallic constituent, reducing the overall energy. The reduction in energy resulting from the secondary diffusion of the reactive constituent promotes the growth of the [1, 1, 1] crystal texture.

Another aspect of the invention involves the angle at which the plasma flux strikes the surface of the device. For pyramidal structures of the desired shape to be formed, it is necessary that the plasma flux strike the surface at a very low angle, that is, the plasma flux should be coming in normal to the surface of the device. On areas of the surface of a device where the plasma flux strikes the surface at an oblique angle, pyramidal structures having flattened tops are more likely to be formed, which will degrade the capacitive performance of the device.

To promote the growth of the coating of the present invention on devices of complex shape, it is therefore necessary to use a cylindrical target during the PVD process to ensure that all surfaces of the device receive plasma flux which is striking that surface on a perpendicular. Although all areas of the device will also have plasma flux striking at an oblique angle, the flux striking at an oblique angle tends to have less energy that that striking on a perpendicular, and therefore has more of an effect on the formation of the desired surface features.

### Description of the Drawings

Figure 1 is a typical prior art coating applied at a normal angle to the surface.

Figure 2 is a typical prior art coating applied at an oblique angle to the surface,

Figure 3a shows a schematic of a PVD apparatus having a flat target

Figure 3b shows a schematic of a PVD apparatus having a cylindrical target

Figure 4 is a coating applied according to one embodiment of the present invention.

Figure 5 is a coating applied according to the preferred embodiment of the present invention.

Figure 6 is a cross sectional view of a coating applied according to the preferred embodiment of the present invention.

Figure 7 is a graph showing capacitance for various trial coating a described herein.

Figure 8 is a graph showing after-potential polarization for various trial coatings described herein.

Figure 9 is a graph showing the optimal performance parameters for obtaining the coating described herein.

### Detailed Description of the Invention

The present invention realizes a performance advantage over typical prior art coatings by achieving a densely packed highly oriented crystalline structure resulting in pyramidal features being exposed on the surface of the coating.

The method of this invention is best practiced using any one of a number of deposition processes, which can generally be described as physical vapor deposition processes, for the deposition of the coating. Various types of physical vapor deposition processes well know in the art include, but are not necessarily limited to magnetron sputtering, cathodic arc, ion beam assisted PVD and LASER ablation PVD, any of which could be used to form the coating described herein. The method of the preferred embodiment is magnetron sputtering.

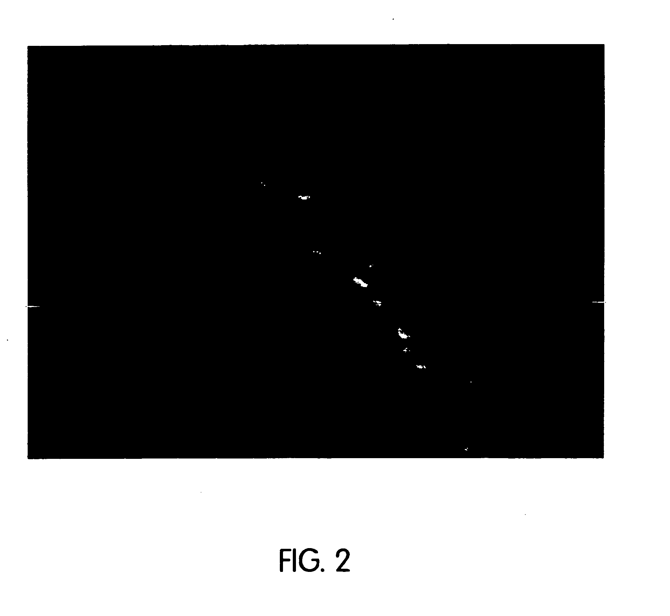

The coating of the present invention is preferably formed using a primary metallic constituent and secondary reactive constituent which will combine with the metallic constituent to promote the growth of a [1, 1, 1] crystal structure. In the preferred embodiment, the primary metallic constituent is titanium, and the secondary reactive constituent is nitrogen, which forms a titanium nitride coating. In the preferred embodiment, approximately 90% plus of the surface of the coating was found to have the desired [1, 1, 1] crystal structure, evidenced by the formation of well-defined pyramidal-shaped structures on the surface. The remainder of the surface is composed of crystals having other structures, such as [2, 0, 0] which results in flat-topped columns, as shown in Figure 2. It has been found that acceptable electrical characteristics can be obtained with surfaces having as low as 80% [1, 1, 1] crystal structure on the surface of the coating.

It has been found that the highest quality surfaces are obtained when the flow ratio of the secondary reactive constituent to the inert gas is approximately 1:1. However, acceptable surfaces have been obtained when the ratio is as high as 1.25: 1, providing a mixture rich in the secondary reactive constituent. It is also possible to use multiple secondary reactive constituents by utilizing various combinations of nitrogen, oxygen and carbon during the deposition process.

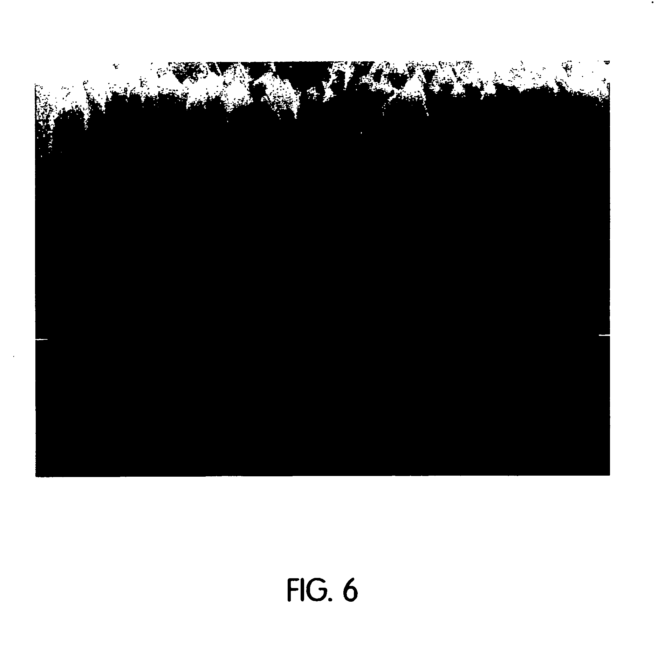

The coating of the present invention is formed by first selecting a substrate temperature such that 0.2 ≤ T_{d}/Tₘ ≤ 0.5 where T_{d} is the temperature of the substrate and Tₘ is the melting point of the primary metallic constituent of the coating. The temperature range described results in a microstructure, known as a Zone 2 microstructure, having a defined columnar structure in which the width of the column is consistent throughout the condensate. This structure can clearly be seen in Figure 6. The surface has an energy high enough to allow some diffusion of the condensate prior to solidification, as well as intermixing of the substrate material and the primary metallic constituent. This surface diffusion allows the material to grow in a preferred crystal orientation and crystal growth is the primary contributor to the structure. As T_{d}/Tₘ, increases, nucleation sites decrease and the amount of surface diffusion increases, which results in decreased inter-columnar porosity.

To achieve the desired temperature T_{d} such that the ratio of T_{d}/Tₘ is in the proper range, power in the deposition system is increased until the proper substrate temperature is achieved. It has been found that a quality surface forms when T_{d}/Tₘ is as low as .2, and that the best quality is achieved when T_{d}/Tₘ is about .25. At values between .25 and .5, the quality of the surface does not appreciably increase, but the rate of formation of the coating increases.

In the preferred embodiment of the invention, the primary metallic constituent should be biocompatible, and the reactive constituent should form a compound with the primary that is electrically conductive, biostable, has anodic and cathodic corrosion resistance and has a cubic crystal structure which can grow in a [1, 1, 1] configuration. Examples of materials are nitrides, oxides and carbides of Ti, Ta, Nb, Hf, Zr, Au, Pt, Pd and W. In the preferred embodiment, titanium is the primary metallic constituent and nitrogen is the reactive constituent. This process will work with a substrate composed of any material capable of reaching a temperature (T_{d} as described above) which permits diffusion and intermixing, such as platinum.

In the preferred embodiment of the invention, titanium is deposited directly onto the substrate in the presence of argon plasma to form an adhesion layer which is inter-diffused with the substrate. The inter-diffusion layer is preferably grown to a thickness of approximately 500 nanometers.

After the growth of the adhesion layer, nitrogen gas is introduced such that the flow ratio of N:Ar is approximately 1:1 to 1.25:1. The partial pressure of N should be kept low such that the ionization percentage of N is also low. This results in a plasma system having high percentages of Ar ions and low percentages of N ions. Since the percentage of ionized N is held low in the processing chamber, it does not react with the Ti until after it has deposited on the substrate or previously condensed surface. The resulting coating is thus composed of titanium nitride.

In addition, for proper growth of the crystalline structure, it is necessary to have the plasma flux strike the surface of the substrate at a normal angle such that the maximum amount of energy is imparted to the substrate to allow the inter-diffusion to occur. Atoms of titanium striking the surface at a normal angle have a greater tendency to "stick" to the surface, while atoms striking at an oblique angle have a greater tendency to skip off of the surface without sticking. This applies both when the pure titanium is being deposited in the inter-diffusion layer, and also when the layer of titanium nitride is being deposited. This tends to reduce or eliminate nucleation sites and allows not only the inner diffusion of the base alloy in the coating but also promotes the growth of the columnar structures.

Because it is desired to have the plasma flux strike the surface at a normal angle, for substrates having a complex shape, it is necessary to utilize a magnetron which approximates a cylindrical shape to ensure that there is a sufficient amount of plasma flux reaching the surface of the substrate at a normal angle, rather than at an oblique angle, such that the sharp peaks on the pyramidal-shaped structures are produced. For these purposes, an actual cylindrical magnetron could be used, or the magnetron could be composed of several planar magnetrons arranged in a circular configuration, such as to surround the substrate.

Several trials were conducted under varying conditions. The trials were conducted using a PVD setup having specific characteristics. Because many different PVD setups may be used, the power required in any specific setup to achieve the same results will vary. All that is required is for the PVD setup to use the amount of power that will cause the temperature of the substrate to reach the desired level, such that the T_{d}/Tₘ ratio is in the proper range. The power required may therefore vary from setup to setup.

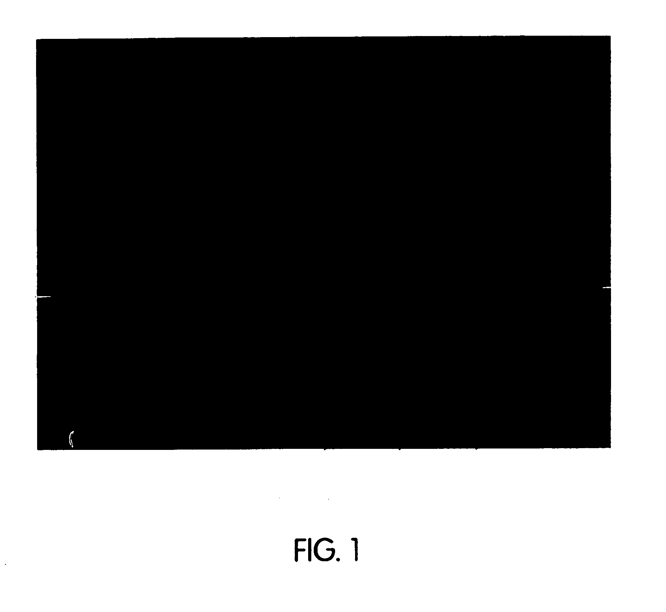

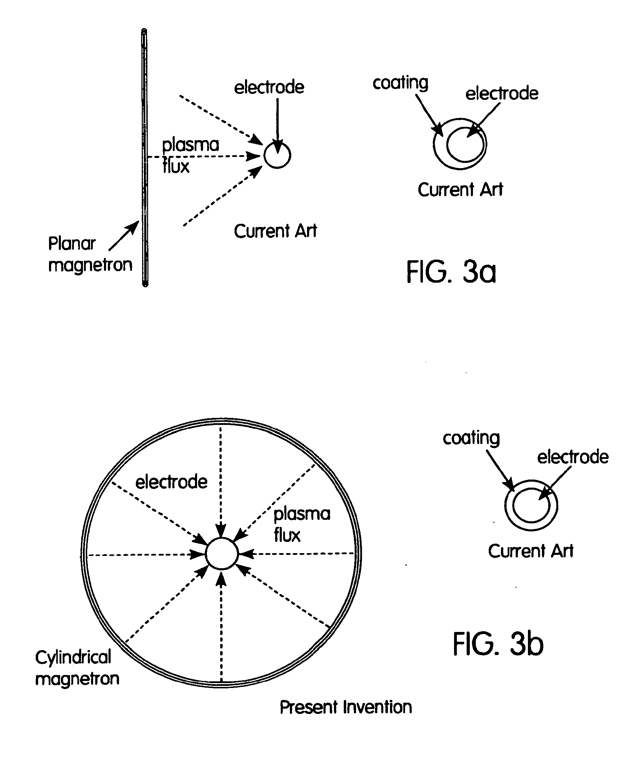

The results of the first trial are shown in Figures 1 and 2, and represent a prior art surface. Titanium was deposited at a power of 1000W with an N:Ar flow ratio of 1:1. The T_{d}/Tₘ ratio in this case is well below the .2 threshold. A PVD setup having a planar magnetron, such as is shown in Figure 3a, was used, although the shape of the magnetron is irrelevant unless device having complex shapes (i.e., not flat) are being coated. The resulting coating shows fibrous columns with a large percentage of internal porosity. Figure 1 shows a coating formed when the plasma flux strikes the surface at an angle normal to the surface, while Figure 2 shows a coating formed when the plasma flux strikes the surface at an oblique angle. Neither the coating in Figures 1 or Figure 2 shows signs of inter-diffusion of the base alloy and the coating, which is expected in a low-energy deposition.

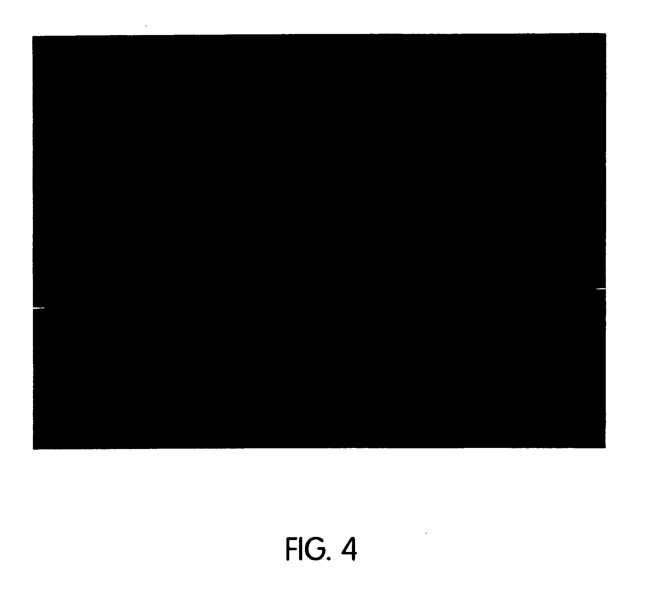

A second trial was conducted according to the present invention. Titanium was deposited at a power of 4000 watts with an N:Ar flow ratio of 1:2. The increase in power to 4000W produced a substrate temperature of about 485°C, resulting in a T_{d}/Tₘ ratio of .29. The resulting coating has continuous columns with a low percentage of porosity and a mix of pyramidal-shaped structures and flat-topped structures. This surface is shown in Figure 4. This process also resulted in the production of a 500 nanometer inter-diffusion later between the coating and the substrate. A though the porosity was reduced and the columnar structures were formed, the lack of pyramidal-shaped structures on the surface having well defined peaks resulted in the coating having a double layer capacitance and after-potential polarization that showed no improvement over prior art coatings. The failure in this case to form the sharp peaks can be attributed to the Ar:N flow ratio of 2:1.

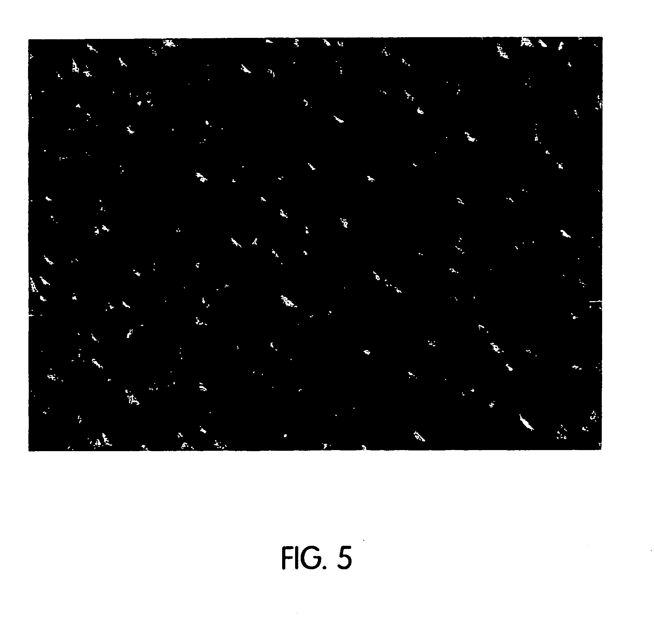

Trial 3 shows a coating formed according to the preferred embodiment of the invention. Here, titanium was deposited at a power of 4000 watts with N:Ar flow ratio of 1:1. As in the previous trial, the T_{d}/Tₘ ratio is .29, but in this case, the flow ratio of N:Ar has been adjusted. The resulting structure has continuous columns with a low percentage of porosity and surface structures having sharp, well defined, pyramidal-shaped peaks. Ideally, the size of the surface structures is about .5 microns. The surface of this coating is shown in Figure 5, clearly showing the pyramidal-shape structure having well defined, sharp peaks. Figure 6 shows a cross-section of the coating showing the continuous columns and low porosity.

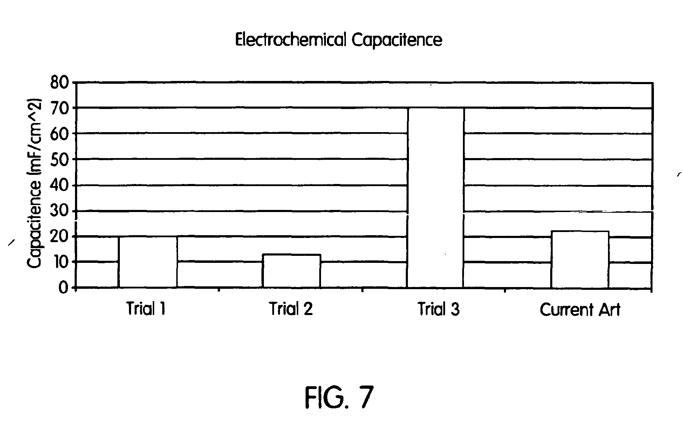

Figure 7 is a graph showing the resulting capacitance of the various surfaces. It can be seen that the electrochemical capacitance for trial 1, which is a prior art type surface is relatively low and for trial 2 the capacitive performance is lower than with the prior art surface. The capacitance for trial 3, showing the preferred embodiment of the invention, approaches 70 µF/cm². The thickness of the coating should range between 2.5 microns and 8 microns in thickness for optimal performance. It has been found that coatings less then 2.5 microns in thickness exhibit a significant drop off in capacitance, while coatings above approximately 7 microns and up to 8 microns reach a maximum capacitance. Note that by increasing the thickness of the coating according to the parameters of the preferred embodiment (Trial 3) the capacitance can be raised to between 90 and 100 µF/cm².

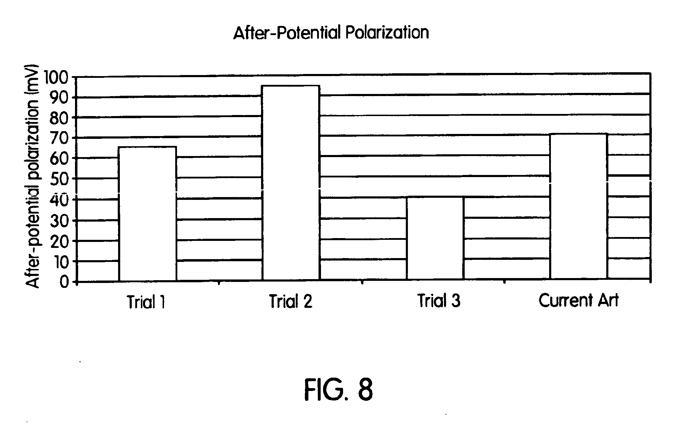

Figure 8 shows the after-potential polarization of the various surfaces. To measure after potential polarization, a sample electrode is immersed in a solution of water and 0.9% molar NaCl along with a counter electrode with an area of greater than 2 times the coated electrode. A periodic signal, such as a square wave, is sent through the sample using a 10 µF capacitive discharge through a 500 ohm resistor. The periodic wave has an amplitude of 5 volts, a pulse length of 1 ms and a frequency of 1 Hz. The resulting waveform is then captured on a data logger. The voltage at 20 ms after the leading edge of the signal is recorded as the "after-potential" polarization. It is desired that the after potential polarization be as low as possible and it can be seen that the preferred embodiment in the invention in Trial 3 shows the desired characteristics.

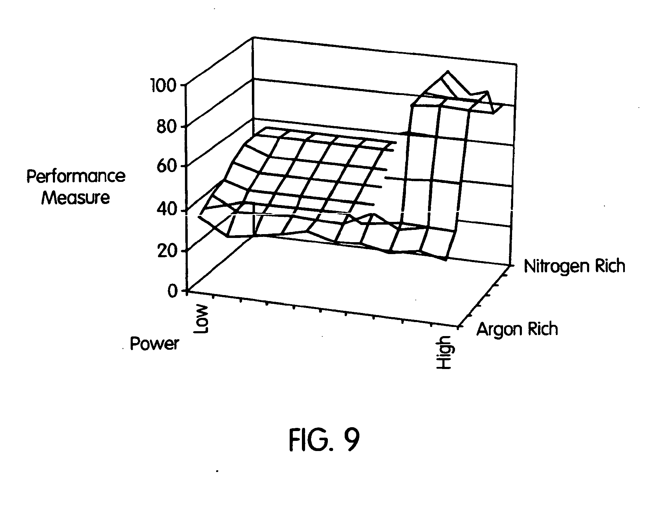

Figure 9 shows a three dimensional graph of power and mixture versus performance, based on various trials. The performance measure has been standardized to reflect desired results for capacitance and after-potential polarization. As can be seen, the dark peak between 60 and 100, representing the best-performing surface, is achieved using high energy with a Ar:N flow ratio of approximately 1:1.

It can clearly be seen from Figures 7-9 that Trial 3 produced the best results, having better capacitance and after-potential polarization performance than prior art surfaces. Trial 2, using an Ar:N flow ratio of 2:1 clearly produces an inferior surface, having characteristics worse than the typical prior art surface.

The key to the invention is the use of a high energy PVD system that is capable of raising the temperature of the substrate to a point where intermixing of the substrate metal and the primary metallic constituent is possible, and, secondly, such that diffusion of the condensate occurs during deposition, such as to reduce nucleation sites in the coating. Additionally, the introduction of the reactive constituent, for example, nitrogen, in the proper ratio, promotes the formation of the [1, 1, 1] crystal structure, resulting a surface having the well-define, sharp, pyramidal-shaped structures.

Note that the specific materials mentioned in the trials, as well as the parameters under which the coatings were formed are meant to be exemplary in nature, and not the exclusive method of producing a coating according to this invention. Other parameters may be used to produce similar results with different materials. Any combination of materials and parameters producing a coating having the desired characteristics is meant to be included within the scope of this invention, which is defined in the claims which follow.

## Claims

1. A method for producing a coating on a substrate using a physical vapor deposition comprising the steps of:
a. providing one or more targets composed of a primary metallic component
b. raising the temperature of said substrate to between 20% and 50% of the melting point of said primary metallic component;
c. positioning said one or more target with respect to said substrate such that a percentage of atoms of said primary component reach said substrate at a normal angle;
d. mixing a secondary reactive component with an inert gas to form a plasma, wherein said secondary reactive component is introduced with the inert gas in a flow ratio of 1:1 to 1.25:1;
e. wherein said primary metallic component and said secondary reactive component react to form a surface having pyramidal crystals defined thereon,
wherein said primary metallic component is allowed to condense on said substrate in the absence of said secondary reactive component to form an intermix layer wherein the atoms of said primary metallic component an the atoms of said substrate are intermixed, and further wherein said secondary reactive component is introduced after the formation of said intermix layer,
the thickness of the coating is 2.5 microns to 8 microns, and
plasma flux strikes the surface of the substrate at a normal angle.

2. The method of claim 1 wherein said secondary reactive component and said inert gas are introduced in an approximate 1:1 ratio.

3. The method of claim 1 or claim 2 wherein said surface crystals have a predominantly [1, 1, 1] structure.

4. The method of any one of the preceding claims wherein said primary metallic component is selected from the group consisting of Ti, Ta, Nb, Hf, Zr, Au, Pt, Pd and W.

5. The method of any one of the preceding claims wherein said secondary reactive component is selected from a group consisting of nitrogen, oxygen and carbon.

6. The method of claim 1 wherein two or more secondary reactive constituents are used, selected from a group consisting of nitrogen, oxygen and carbon.

7. The method of any one of the preceding claims wherein the temperature of said substrate is raised to approximately 25% of the melting point of said primary metallic component.

8. The method of any one of the preceding claims wherein the target is cylindrical.

9. The method of claim 1 where multiple targets of said primary metallic component are used, and said multiple targets are arranged in a circular pattern with said substrate at the center.

10. An implantable medical electrode having a coating thereon comprising a zone 2 microstructure composed of a primary metallic component and a secondary reactive component, said surface having crystals with a [1, 1, 1] structure defined thereon, wherein an intermix layer has been formed between the surface of said electrode and said coating, said intermix layer composed primarily of said primary metallic component intermixed at the surface of said electrode with atoms of the material of which said electrode is composed the coating and intermix layer formable by the method of any one of claims 1 to 9, such that the thickness of the coating is 2.5 microns to 8 microns.

11. The implantable medical electrode of claim 10 wherein said coating is a nitride of an element selected from the group consisting of Ti, Ta, Nb, Hf, Zr, Au, Pt, Pd and W.

## Patentansprüche

1. Herstellungsverfahren für eine Beschichtung auf einem Substrat durch physikalische Gasphasenabscheidung, umfassend die Schritte
a. Bereitstellen eines oder mehrerer Ziele, bestehend aus einem metallischen Primärbestandteil
b. Erhöhen der Temperatur des Substrats auf bis zu zwischen 20% und 50% des Schmelzpunkts des metallischen Primärbestandteils;
c. Positionieren des einen oder der mehreren Ziele gegenüber dem Substrat, so dass ein Prozentsatz Atome des Primärbestandteils das Substrat in einem Normalwinkel erreichen;
d. Vermischen eines reaktiven Sekundärbestandteils mit einem Edelgas, um ein Plasma zu Bilden, wobei der reaktive Sekundärbestandteil in einem Fließverhältnis von 1:1 bis 1,25:1 mit dem Edelgas eingeführt wird;
e. wobei der metallische Primärbestandteil und der reaktive Sekundärbestandteil reagieren, um eine Oberfläche mit darauf definierten pyramidalen Kristallen zu bilden,
wobei dem metallischen Primärbestandteil ermöglicht wird, auf dem Substrat in der Abwesenheit des reaktiven Sekundärbestandteils zu kondensieren, um eine Intermixschicht zu bilden, wobei die Atome des metallischen Primärbestandteils und die Atome des Substrats untereinander vermischt sind, und wobei zudem der reaktive Sekundärbestandteil nach der Bildung der Intermix-Schicht eingeführt wird,
die Stärke der Beschichtung 2,5 µm bis 8 µm ist, und
ein Plasmafluss die Oberfläche des Substrats in einem Normalwinkel trifft.

2. Herstellungsverfahren gemäß Anspruch 1, wobei der reaktive Sekundärbestandteil und das Edelgas in einem ungefähren 1:1-Verhältnis eingeführt werden.

3. Herstellungsverfahren gemäß Anspruch 1 oder Anspruch 2, wobei die Oberflächenkristalle vorwiegend eine [1, 1, 1]-Struktur aufweisen.

4. Herstellungsverfahren gemäß irgendeinem der vorherigen Ansprüche, wobei der metallische Primärbestandteil ausgewählt ist aus der Gruppe Ti, Ta, Nb, Hf, Zr, Au, Pt, Pd und W.

5. Herstellungsverfahren gemäß irgendeinem der vorherigen Ansprüche, wobei der reaktive Sekundärbestandteil ausgewählt ist aus der Gruppe Stickstoff, Sauerstoff und Kohlenstoff.

6. Herstellungsverfahren gemäß Anspruch 1, wobei zwei oder mehr reaktive Sekundärbestandteile verwendet werden, ausgewählt aus der Gruppe Stickstoff, Sauerstoff und Kohlenstoff.

7. Herstellungsverfahren gemäß irgendeinem der vorherigen Ansprüche, wobei die Temperatur des Substrats bis zu ungefähr 25% des Schmelzpunkts des metallischen Primärbestandteils erhöht wird.

8. Herstellungsverfahren gemäß irgendeinem der vorherigen Ansprüche, wobei das Ziel zylinderförmig ist.

9. Herstellungsverfahren gemäß Anspruch 1, wobei mehrere Ziele des metallischen Primärbestandteils verwendet werden, und wobei die mehreren Ziele in einem kreisförmigen Muster mit dem Substrat in der Mitte angeordnet sind.

10. Implantierbare medizinische Elektrode mit einer Beschichtung darauf, umfassend eine Zone-2-Mikrostruktur, zusammengesetzt aus einem metallischen Primärbestandteil und einem reaktiven Sekundärbestandteil, wobei die Oberfläche Kristalle mit einer [1, 1, 1]-Struktur darauf definiert aufweist, wobei eine Intermix-Schicht zwischen der Oberfläche der Elektrode und der Beschichtung gebildet worden ist, zusammengesetzt vorwiegend aus dem metallischen Primärbestandteil und an der Oberfläche der Elektrode vermischt mit Atomen des Materials aus dem die Elektrode zusammengesetzt ist, wobei die Beschichtung und die Intermix-Schicht mit dem Verfahren aus irgendeinem der Ansprüche 1 bis 9 gebildet werden können, so dass die Stärke der Beschichtung 2,5 µm bis 8 µm ist.

11. Implantierbare medizinische Elektrode gemäß Anspruch 10, wobei die Beschichtung ein Nitrid eines Elements ist, ausgewählt aus der Gruppe Ti, Ta, Nb, Hf, Zr, Au, Pt, Pd und W.

## Revendications

1. Un procédé de production d'un revêtement sur un substrat au moyen d'un dépôt physique en phase vapeur comprenant les opérations suivantes :
a. la fourniture d'une ou plusieurs cibles composées d'un composant métallique primaire,
b. l'élévation de la température dudit substrat à entre 20% et 50% du point de fusion dudit composant métallique primaire,
c. le positionnement desdites une ou plusieurs cibles par rapport audit substrat de sorte qu'un pourcentage des atomes dudit composant primaire atteigne ledit substrat à un angle normal,
d. le mélange d'un composant réactif secondaire avec un gaz inerte de façon à former un plasma, où ledit composant réactif secondaire est introduit avec le gaz inerte dans un rapport d'écoulement de 1:1 à 1.25:1,
e. où ledit composant métallique primaire et ledit composant réactif secondaire réagissent de façon à former une surface possédant des cristaux pyramidaux définis sur celle-ci,
où ledit composant métallique primaire est autorisé à se condenser sur ledit substrat en l'absence dudit composant réactif secondaire de façon à former une couche de mélange où les atomes dudit composant métallique primaire et les atomes dudit substrat sont intermélangés, et où en outre ledit composant réactif secondaire est introduit après la formation de ladite couche de mélange,
l'épaisseur du revêtement est de 2,5 microns à 8 microns, et
le flux de plasma frappe la surface du substrat à un angle normal.

2. Le procédé selon la revendication 1 où ledit composant réactif secondaire et ledit gaz inerte sont introduits dans un rapport d'environ 1:1.

3. Le procédé selon la revendication 1 ou 2 où lesdits cristaux de surface possèdent une structure principalement [1, 1, 1].

4. Le procédé selon l'une quelconque des revendications précédentes où ledit composant métallique primaire est sélectionné dans le groupe se composant de Ti, Ta, Nb, Hf, Zr, Au, Pt, Pd et W.

5. Le procédé selon l'une quelconque des revendications précédentes où ledit composant réactif secondaire est sélectionné dans un groupe se composant d'azote, d'oxygène et de carbone.

6. Le procédé selon la revendication 1 où deux ou plus composants réactifs secondaires sont utilisés, sélectionnés dans un groupe se composant d'azote, d'oxygène et de carbone.

7. Le procédé selon l'une quelconque des revendications précédentes où la température dudit substrat est élevée à environ 25% du point de fusion dudit composant métallique primaire.

8. Le procédé selon l'une quelconque des revendications précédentes où la cible est cylindrique.

9. Le procédé selon la revendication 1 où plusieurs cibles dudit composant métallique primaire sont utilisées et lesdites plusieurs cibles sont agencées selon un motif circulaire avec ledit substrat au centre.

10. Une électrode médicale implantable possédant un revêtement sur celle-ci comprenant une microstructure de zone 2 composée d'un composant métallique primaire et d'un composant réactif secondaire, ladite surface possédant des cristaux avec une structure [1, 1, 1] définie sur celle-ci, où une couche de mélange a été formée entre la surface de ladite électrode et ledit revêtement, ladite couche de mélange étant composée principalement dudit composant métallique primaire intermélangé à la surface de ladite électrode avec des atomes du matériau dont ladite électrode est composée, le revêtement et la couche de mélange étant formables par le procédé selon l'une quelconque des revendications 1 à 9, de sorte que l'épaisseur du revêtement soit de 2,5 microns à 8 microns.

11. L'électrode médicale implantable selon la revendication 10 où ledit revêtement est un nitrure d'un élément sélectionné dans le groupe se composant de Ti, Ta, Nb, Hf, Zr, Au, Pt, Pd et W.
